# EUROPÄISCHE PATENTSCHRIFT

(11) **EP 0 303 983 B1**
(45) Veröffentlichungstag und Bekanntmachung des Hinweises auf die Patenterteilung: **22.09.1993**
(21) Anmeldenummer: 88113124.7
(22) Anmeldetag: 12.08.1988
(51) Int. Cl.: C07K 7/10, C07K 17/02, C07K 3/18, C07K 15/00, G01N 33/68, C07K 7/06, C07K 7/08

(54) **Synthetische Peptide, gegen diese gerichtete Antikörper und deren Verwendung**
Synthetic peptides, antibodies directed against them and their use
Peptides synthétiques, les anticorps contraires et leur utilisation

(30) Priorität: 19.08.1987 DE 3727610
(43) Veröffentlichungstag der Anmeldung: 22.02.1989
(73) Patentinhaber: BEHRINGWERKE Aktiengesellschaft, 35001 Marburg (DE)
(72) Erfinder: Pelzer, Hermann, Dr., D-3553 Cölbe (DE); Stüber, Werner, Dr., D-3551 Lahntal (DE)

(56) Entgegenhaltungen:
- Keine Entgegenhaltungen.

## Beschreibung

Die Erfindung bezieht sich auf synthetische Peptide, die Verwendung dieser Peptide zum Immunisieren eines Tieres und zur Reinigung von spezifischen Antikörpern gegen die genannten Peptide, auf Antikörper gegen diese Peptide und die Verwendung dieser Antikörper.

Der Organismus verfügt über zwei im Gleichgewicht stehende Systeme, um sich sowohl vor Blutverlust als auch vor Thrombosen zu schützen: das Gerinnungssystem und das fibrinolytische System. Das Zusammenspiel zwischen beiden Systemen gewährleistet, daß zunächst zur Blutstillung unlösliche Fibrinpolymere entstehen, die während der Wundheilung durch den lytischen Vorgang der Fibrinolyse wieder abgebaut werden.

Plasmin und Thrombin stellen die Schlüsselenzyme beider Systeme dar. Unter physiologischen Bedingungen steht das dynamische Gleichgewicht zwischen dem Fibrinolyse- und Gerinnungssystem unter Kontrolle der thrombolytischen Aktivität von Plasmin und der thromboplastischen Aktivität von Thrombin.

Thrombin ist eine typische Serinprotease, die in Form eines inaktiven Vorläufers, des Prothrombins, synthetisiert wird. Die Aktivierung des Prothrombins beruht auf der Proteolyse durch den Gerinnungsfaktor Xa, der innerhalb der Gerinnungskaskade eine zentrale Position einnimmt. Faktor X selbst hat insofern eine Sonderstellung inne, als er sowohl über den exogenen, als auch über den endogenen Weg der Gerinnung aktiviert werden kann. Aktivierter Faktor X (Faktor Xa) aktiviert Prothrombin, indem er das Prothrombinmolekül spezifisch ab der dem Tetrapeptid Ile-Glu-Gly-Arg folgenden Peptidbindungen spaltet. Durch diese Spaltung entsteht einerseits Thrombin, andererseits in äquimolarer Konzentration das Prothrombinfragment F₁₊₂. Da aus jedem gespaltenen Prothrombinmolekül ein Molekül Thrombin und ein Molekül Prothrombinfragment F₁₊₂ entstehen, läßt eine Bestimmung des Prothrombinfragments F₁₊₂ im Blut oder Plasma einen direkten Rückschluß auf die Gerinnungskapazität zu, die von der im Blut oder Plasma enthaltenen Thrombinkonzentration abhängig ist.

Im Stand der Technik ist die Quantifizierung von Thrombin bzw. Prothrombinfragmenten F₂/F₁₊₂ unter Verwendung von Radioimmunoassays bekannt. Die hierfür notwendigen Antiseren werden erzeugt, indem aus gereinigten Prothrombinmolekülen gewonnene Prothrombinfragmente F₂ und F₁₊₂ zum Immunisieren von Tieren verwendet werden. Aus den so erhaltenen Rohantiseren werden die spezifischen Antikörper durch immunadsorptive Reinigung an immobilisiertem Prothrombin und den entsprechenden Fragmenten angereichert. Diese Antikörperpräparationen eignen sich zur Bestimmung der Prothrombinfragmente F₂/F₁₊₂, ermöglichen keine vollständige Unterscheidung zwischen intaktem, ungespaltenem Prothrombin und durch Spaltung freigesetzen Prothrombinfragmenten F₂/F₁₊₂. Darüberhinaus erlaubt die relativ geringe Spezifität dieser Antikörperpräparation eine Bestimmung des Antigens ausschließlich unter Verwendung von Radioimmunoassays (RIA), die in der Praxis nur unter Beachtung der durch die Strahlenschutzverordnung festgelegten Bedingungen durchführbar sind und deshalb einen relativ großen technischen und finanziellen Aufwand erfordern. Schließlich müssen mit radioaktiven Isotopen markierte Antikörper immer wieder frisch bereitgestellt werden, da das für die radioaktive Markierung von Proteinen üblicherweise verwendete Isotop ¹²⁵Jod eine Halbwertszeit von nur rund 2 Monaten hat.

Der vorliegenden Erfindung lag daher die Aufgabe zugrunde, Antigene zur Verfügung zu stellen, die zur Bildung spezifischer Antikörper gegen die Prothrombinfragmente F₂/F₁₊₂ führen und somit die rasche und exakte Bestimmung des Gehaltes dieser Fragmente in biologischen Flüssigkeiten ermöglichen.

Diese Aufgabe wird erfindungsgemäß durch synthetische Peptide gelöst, die Aminosäuresequenzen aufweisen, die teilweise der Aminosäurensequenz von Prothombin entsprechen und antigen sind.

Gegenstand der Erfindung sind deshalb Peptide, die Aminosäurensequenzen aufweisen, die teilweise dem durch die FXa-Spaltung von Thrombin entstehenden carboxyterminalen Ende der Fragmente F₂/F₁₊₂ entsprechen, dadurch gekennzeichnet, daß sie die Aminosäuresequenz H-Gly-Asp-Glu-Glu-Gly-Val-Trp-Cys-Tyr-Val-Ala-Gly-Lys-Pro-Gly-Asp-Phe-Gly-Tyr-Cys-Asp-Leu-Asn-Tyr-Cys-Glu-Glu-Ala-Val-Gln-Glu-Glu-Thr-Gly-Asp-Gly-Leu-Asp-Glu-Asp-Ser-Asp-Glu-Glu-Arg-Ala-Ile-Glu-Gly-Arg-Oh- ganz oder teilweise mindestens aber die vier carboxyterminalen Aminosäuren enhalten.

Zur Herstellung der erfindungsgemäßen Peptide eignen sich gängige Methoden, beispielsweise die Festphasenpeptidsynthese nach Merrifield (G. Barany u. R. B. Merrifield: "Solid-Phase Peptide Synthesis" in E. Gross und J. Meienhofer: The Peptides, Analysis, Synthesis, Biology, Academic Press, Inc. 1980) sowie herkömmliche Synthesestrategien, die den Aufbau der Peptide in Form löslicher Peptidsegmente ausführen. Besonders bevorzugt wurde das Peptid der Struktur H-Cys(SH)-Leu-Asp-Glu-Asp-Ser-Asp-Glu-Glu-Arg-Ala-Ile-Glu-Gly-Arg an der festen Phase hergestellt. Als temporäre Schutzgruppe wurde die Fmoc-Gruppe, als permanente Schutzgruppe für Asp und Glu die O-t-Bu-Schutzgruppe, für Ser die t-Bu, für Arg die Mtr-Gruppe und Cys die tert.-Butylmercaptogruppe eingesetzt. Die Immobilisierung der C-terminalen Aminosäuren erfolgte über p-Alkoxybenzylestergruppe, die an 1 % quervernetztes Polystyrol gebunden waren. Der Aufbau der Peptide erfolgte unter repetitiver Abspaltung der temporären Schutzgruppe und Kondensation der folgenden, geschützten Aminosäure mit einem Kondensationsmittel, bevorzugt einem Carbodiimid. Die Abspaltung der Peptide vom Harz erfolgte acidolytisch unter gleichzeitiger Freilegung der Seitenkettenfunktionen. Gegebenenfalls freizulegende Sulfhydrylfunktionen werden nach dem Stand der Technik mittels Tri-n-Butylphosphin entschützt. Die Reinigung der Peptide erfolgte über Ionenaustauschchromatographie, RP-HPLC und Gelpermeationschromatographie. Die korrekte Zusammensetzung der Peptide wurde durch Aminosäureanalytik bestätigt.

Die Verwendung von synthetischen Peptiden als Antigene bei der Immunisierung von Tieren führt zur Erzeugung von speziell gegen das in diesem Peptid exponierte Hapten gerichteten Antikörpern. Die so erzeugten Antikörper sind daher spezifisch für jeweils eine einzige Antikörperbindungsstelle des gesamten Proteins, aus dem die Peptidsequenz abgeleitet worden ist. Die Verwendung von synthetischen Peptiden hat gegenüber der Verwendung der natürlichen gereinigten Prothrombinfragmente F₂/F₁₊₂ weiterhin zwei gravierende Vorzüge; synthetische Peptide lassen sich in großem Maßstab und in hoher Reinheit herstellen, so daß das aufwendige Isolieren und Reinigen der natürlichen Prothrombinfragmente entfällt. Während die Reinigung synthetischer Peptide von Synthesenebenprodukten gut etabliert ist, führen auch technisch aufwendige Anreicherungs- und Reinigungsverfahren für natürliche Prothrombinfragmente stets zu Präparationen, die einen, wenngleich unbestimmbar kleinen, so doch als Antigen wirksamen Anteil unerwünschter Peptide enthalten.

Erfindungsgemäß wird ein Peptid, dessen Aminosäuresequenz ganz oder teilweise der Aminosäuresequenz von Prothrombin entspricht, nach einer der gängigen Peptidsyntheseverfahren, z. B. der Merrifield-Synthese, hergestellt. Bei der Auswahl der entsprechenden Sequenz werden möglichst die Bereiche ausgewählt, von denen wegen ihrer Lokalisierung im Protein und/oder der Antigenizität des exponierten Epitops eine hochantigene Wirkung vorhergesagt werden kann. Das synthetische Peptid ist dann als Antigen wirksam, so daß durch eine Immunisierung eine Immunantwort ausgelöst wird.

Der aktivierte Faktor X (Xa) spaltet das Prothrombinmolekül an der Erkennungssequenz Ile-Glu-Gly-Arg. Das Vorkommen dieses Tetrapeptides ist bisher nur in humanem und bovinem Prothrombin nachgewiesen worden. Die Seltenheit dieses Tetrapeptides prädestiniert es zur Verwendung als spezifisches Merkmal des Prothrombinmoleküls.

Die Spaltung des Prothrombins durch aktivierten Faktor X erfolgt im Anschluß an das Arginin der Sequenz Ile-Glu-Gly-Arg. Durch Faktor Xa wird also ein neuer Carboxyterminus generiert, der im terminalen Bereich die beiden Aminosäuren Glu und Arg enthält, die einen sehr hohen Antigenizitätsindex aufweisen. Peptide oder Polypeptide, die C-terminal die Erkennungssequenz der Faktor Xa-Spaltstelle enthalten, eignen sich besonders gut zur Immunisierung.

Es hat sich überraschenderweise gezeigt, daß Antikörper, die gegen ein Peptid oder Polypeptid gerichtet sind, das an seinem C-terminalen Ende die Sequenz des Tetrapeptides der Erkennungssequenz der Faktor Xa-Protease enthält, ausschließlich spezifisch mit abgespaltenen Prothrombinfragmenten reagieren, nicht jedoch mit dem intakten Prothrombinmolekül.

Das durch Einwirkung des Faktors Xa auf Prothrombin entstandene Fragment, das C-terminal die Faktor Xa-Erkennungssequenz trägt, ist 273 Aminosäuren lang. Zur Immunisierung eignet sich sowohl das ganze Polypeptid, als auch Teilsequenzen dieses Peptides, die aber nach wie vor am C-Terminus die Faktor Xa-Erkennungssequenz aufweisen sollen. Eine besonders bevorzugte Ausführungsform sieht die Verwendung eines Tetradekapeptides vor, beispielsweise mit der Sequenz Leu-Asp-Glu-Asp-Ser-Asp-Glu-Glu-Arg-Ala-Ile-Glu-Gly-Arg-OH. Für beide Fälle ist lediglich wichtig, daß die carboxyterminale Sequenz des Moleküls exponiert ist und zur Immunisierung führt.

In den Fällen, in denen das Peptid nicht dem Auslösen einer Immunantwort dienen soll, sondern die Funktion dieses Peptides nur sein soll, von bereits bestehenden Antikörpern erkannt zu werden, sind kürzere Peptide ausreichend. Eine zweckmäßige Ausführungsform beruht dabei auf einem Oktapeptid der Sequenz Glu-Glu-Arg-Ala-Ile-Glu-Gly-Arg-OH.

In Anbetracht der für die Peptide vorgesehenen Verwendung ist es sinnvoll, Aminosäuren mit reaktiven Seitengruppen so in die Peptide einzuführen, daß sie die Struktur des Haptens nicht beeinflussen. Zweckmäßigerweise wird aus diesem Grund N-terminal als eine weitere Aminosäure Cystein angefügt, deren freie SH-Gruppe zur Kopplung via Thioether an viele Träger geeignet ist. Bevorzugt wird z. B. das durch das obenangegebene Peptid repräsentierte Antigen in Form des Pentadecapeptides Cys(SH)-Leu-Asp-Glu-Asp-Ser-Asp-Glu-Glu-Arg-Ala-Ile-Glu-Gly-Arg zur Verfügung gestellt.

Die Herstellung des zur Immunisierung eingesetzten Peptides kann sowohl auf üblichem Wege durch chemische Synthese geschehen, als auch durch Reinigung eines gentechnologisch bereitgestellten Polypeptides. Denkbar wäre z. B. die gentechnische, von einem starken Promotor gesteuerte Synthese des Prothrombinfragmentes F₂ oder F₁₊₂ in E.coli oder auch die gentechnologische Synthese eines über die Faktor Xa-Spaltstelle hinausgehenden Peptides oder Peptides, das anschließend in vitro durch ebenfalls gentechnisch gewonnenen Faktor Xa gespalten wird, was zur Freisetzung des gewünschten antigenen Carboxyterminus führt.

Peptide, die zur Immunisierung eingesetzt werden sollen oder solche, die Verwendung als Immunadsorbens finden sollen, werden sinnvollerweise an ein Trägermolekül gekoppelt. Übliche, vielfach verwendete Trägermoleküle sind z. B. Rinderserumalbumin, Ovalbumin und Polysaccharide. In einer bevorzugten Ausführungsform wird das Peptid oder Polypeptid an das Hämocyanin einer marinen Napfschnecke, das Keyhole Limpet Hemocyanin, gebunden.

Bei Verwendung der erfindungsgemäßen synthetischen Peptide als Immunadsorbentien empfiehlt sich die Kopplung an Materialien, die sich zum Bereitstellen fester Matrizes eignen. Eine bevorzugte Ausführungsform sieht die Kopplung kurzer Peptide, z. B. des obenerwähnten Oktapeptides, an bromcyanaktivierte Sepharose vor.

Die Immunisierung geeigneter Tiere mit trägergebundenen Peptiden führt reproduzierbar zur Bildung von Antikörpern. Eine bevorzugte Tierspezies für Immunisierung und Antikörpergewinnung ist hierbei das Kaninchen, da sich hier Zucht- und Pflegeaufwand im Verhältnis zum erhältlichen Blutvolumen im günstigsten Verhältnis befinden.

Aus einem solchen, erfindungsgemäß mit synthetischen Peptiden im Tier erzeugten Antiserum kann die für spezifische Tests relevante Immunglobulinfraktion durch übliche immunadsorptive Methoden angereichert werden. Bevorzugt ist es in diesem Falle jedoch, als Material für eine solche zur Immunadsorption eingesetzte Matrix ebenfalls ein an einen Träger gekoppeltes Peptid zu verwenden, das die gleiche antigene Determinante wie das zur Immunisierung eingesetzte Peptid aufweist. Das zur immunadsorptiven Reinigung verwendete Peptid kann dabei erheblich kürzer sein; Voraussetzung zur Verwendung in der immunadsorptiven Reinigung des gewünschten Antikörpers ist lediglich, daß die von diesem kürzeren Polypeptid gebildete antigene Determinante vom gewünschten Antikörper erkannt und effektiv gebunden wird.

Das für die immunadsorptive Reinigung der Antikörper verwendete Peptid kann z. B. ein Oktapeptid sein, bevorzugt das Peptid H-Glu-Glu-Arg-Ala-Ile-Glu-Gly-Arg.

Erfindungsgemäß werden durch Immunisieren mit synthetischen Peptiden im tierischen System Antikörper induziert und durch Immunadsorption gereinigt. Diese Antikörper reagieren spezifisch mit den zur Immunisierung und Reinigung verwendeten Peptiden. In Abhängigkeit von der Sequenz des verwendeten Peptids binden diese Antikörper nur an die Fragmente F₂ und F₁₊₂.

Durch Auswahl entsprechender Peptide als Immunadsorbentien können Antikörper selektioniert werden, die spezifisch mit der antigenen Determinante des Prothrombins, die der Sequenz der Faktor Xa-Spaltstelle dieses Moleküls entspricht, reagieren; in dem bevorzugten Fall, daß Peptide, die C-terminal die Faktor Xa-Erkennungssequenz aufweisen, sowohl zur Immunisierung als auch zur immunadsorptiven Reinigung verwendet werden, werden Antikörper gegen diese Sequenz angereichert, die jedoch nicht mit intaktem, nativen Prothrombin reagieren, da im nativen Prothrombinmölekül die Faktor Xa-Spaltstelle entweder nicht stark genug exponiert ist oder nicht die zur antigenen Erkennung erforderliche höhere Struktur aufweist.

Die erfindungsgemäß gewonnenen Antikörper können für eine Reihe verschiedenartig konzipierter Immunoassays eingesetzt werden. Zweckmäßigerweise werden sie dazu an einen festen Träger gekoppelt, bevorzugt aber durch Adsorption an Polystyrolröhrchen immobilisiert. Die für die folgenden Immunoassays vorbereiteten Röhrchen können anschließend luftdicht verschlossen bei 4° C gelagert werden.

Die erfindungsgemäße Bestimmung des Gehaltes an Prothrombinfragmenten F₂/F₁₊₂ erfolgt durch Vorinkubation der Probe mit solcher Art immobilisierten Antikörpern, wobei die Konzentration der durch die immobilisierten Antikörper gebundenen Fragmente F₂/F₁₊₂ durch eine nachfolgende Inkubation mit einem zweiten Antikörper detektiert wird. Dieser zweite Antikörper muß eine Eigenschaft aufweisen, die meßbar ist, z. B. die Fähigkeit zur Umsetzung oder Bindung eines chromogenen Substrates.

Zweckmäßigerweise wird dieser zweite Antikörper entweder mit einem Markerenzym gekoppelt, bevorzugt Peroxidase. Wahlweise kann der zweite Antikörper jedoch auch entweder mit einem fluoreszierenden Molekül, z. B. Flouresceinisothiocyanat, oder aber einer radioaktiven Markierung versehen werden.

Die Bestimmung der Prothrombinfragmente F₂/F₁₊₂ kann auch durch gleichzeitige Inkubation der Probe, bevorzugt von Plasma und markiertem Antikörper mit den immobilisierten Antikörpern erfolgen. Darüber hinaus ist ein kompetitives Bestimmungsverfahren möglich, wobei markierte und unmarkierte Prothrombinfragmente F₂/F₁₊₂ um die Bindungsstelle der immobilisierten Antikörper konkurrieren.

Der auf diese Weise bestimmte Gehalt an Prothrombinfragmenten F₂/F₁₊₂ läßt eine Aussage über den Aktivierungsgrad des Prothrombins zu.

Die Beispiele erläutern die Erfindung. In den Beispielen werden folgende Abkürzungen verwendet:
- FPA: Fibrinopeptid A
- ELISA: Enzymimmunoassay (enzyme linked immunosorbent assay)
- RIA: Radioimmunoassay
- KLH: Keyhole Limpet hemocyanin (Haemocyanin einer marinen Napfschnecke)
- PBS: Phosphatgepufferte Kochsalzlösung (phosphate buffered saline)
- Tris: Tris (hydroxymethyl) aminomethan
- EDTA: Äthylendinitrilotetraessigsäure
- OD: Extinktion (optical density)
- Asp: L-Asparaginsäure
- Ala: L-Alanin
- Arg: L-Arginin
- Gly: Glycin
- Glu: L-Glutaminsäure
- Ile: L-Isoleucin
- Ser: L-Serin
- Cys(SH): L-Cystein
- Fmoc: 9-Flourenylmethyloxycarbonyl
- O-t-Bu: tert.-Butylester
- t-Bu: tert.-Butylether
- Mtr: 4-Methoxy-2,3,6-trimethylphenylsulfonyl
- DMF: Dimethylformamid
- RP-HPLC: reversed phase high performance liquid chromatography
- GMBS: gamma-Maleimidobuttersäurehydroxysuccinimidester

### Beispiel 1

### Herstellung eines Antigens zum Immunisieren

### a) Peptidsynthese des Pentadecapeptides H-Cys(SH)-Leu-Asp-Glu-Asp-Ser-Asp-Glu-Arg-Ala-Ile-Glu-Gly-Arg

Die Synthese des Peptids erfolgte unter Anwendung eines halbautomatischen Peptidsynthesizers. 1 g Fmoc-Arg (Mtr)-p- alkoxybenzylesterharz wurde mit 15 ml 20 % Piperidin/DMF (v/v) entschützt und daraufhin mit DMF und Isopropanol mehrfach gewaschen. Es wurden 1 mMol Fmoc-Gly (3facher Überschuß) und 203 mg HOBt in 15 ml DMF gelöst zugesetzt. Nach Zugaben von 1,1 ml einer 1 M Diisopropylcarbodiimidlösung (Dichlormethan) wurde die Kopplung für 1,5 Stunden durchgeführt. Durch Waschen mit DMF und Isopropanol wurden überschüssige Reagentien entfernt. Dieses Kopplungsschema wurde bis zur N-terminalen Aminosäure beibehalten. Als letzte Aminosäure wurde eine Boc-geschützte Aminosäure eingesetzt. Jeder Kopplungsschritt wurde durch einen Ninhydrintest auf Vollständigkeit geprüft. 1,06 g Harz wurden mit 2,5 ml Thioanisol, 2,5 ml Ethandithiol und 15 ml Triflouressigsäure 4 Stunden bei 35° C gerührt und abfiltriert. Die saure Lösung wurde in Ether gegossen, das ausgefallene Peptid abfiltriert und an einer Sephadex® G 25-Säule (3 x 100 cm, 0,5 % Essigsäure) chromatographiert. Der Peptidpool wurde lyophilisiert. Ausbeute: 230 mg Peptid.

### b) Freisetzung der Sulfhydrylgruppe

70 mg des Peptids wurden in 7 ml Trifluorethanol und 350 µl Wasser gelöst und der pH-Wert mit N-Methylmorpholin auf 7,3 eingestellt. Das Reaktionsgefäß wurde mit Stickstoff gespült und mit 40 µl n-Tributylphosphin versetzt. Es wurde 1 Stunde bei Raumtemperatur gerührt, mit 50 ml Wasser verdünnt und der pH-Wert auf 4,0 gestellt. Die Wasserphase wurde dreimal mit 10 ml Diethylether extrahiert, auf 10 ml ankonzentriert und an Sephadex® G 25 (3 x 100 cm; 0,5 % Essigsäure) gereinigt. Ausbeute: 55 mg Peptid.

### c) Konjugatherstellung

30 mg Keyhole Limpet Hemocyanin wurden in 0,05 mMol Natriumphosphatpuffer pH 8,0 gelöst und mit 3 mg GMBS 1 Stunde lang aktiviert. Das Protein wurde an einer Sephadex^{®} G 50-Säule (2 x 30 cm) (0,1 mol Natriumphosphat; 0,5 mMol EDTA pH 6,0) chromatographiert. Der Proteinpool wurde auf 6 ml eingeengt und mit 30 mg des Sulfhydrylgruppenhaltigen Peptids 1 Stunde lang inkubiert. Ausbeute nach Dialyse und Lyophilisation: 38 mg Peptidkonjugat.

### Beispiel 2

### Immunisieren von Kaninchen

5 Kaninchen wurden mit jeweils 2 mg Antigen pro Tier über einen Zeitraum von 8 Wochen immunisiert; die Applikationen des Peptid-KLH-Konjugats erfolgten subcutan und intravenös. Danach wurden die Tiere entblutet, die gewonnenen Rohantiseren gepoolt und mit Konservierungsmittel stabilisiert. Ausbeute: 850 ml Antiserum.

### Beispiel 3

### Herstellen von Immunadsorbentien

Für die affinitätschromatographische Reinigung der Rohantiseren wurden ca. 20 mg Oktapeptid der Sequenz H-Glu-Glu-Arg-Ala-Ile-Glu-Gly-Arg-OH (wie in Beipiel 1a hergestellt) an einer Festphase kovalent immobilisiert. Die Kopplungsreaktion erfolgte mit Bromcyan-aktivierter Sepharose nach einem beschriebenen Verfahren (Axen, R. et al., Nature, 214, 1302, 1967). Anschließend wurde das Immunadsorbens jeweils mit phosphatgepufferter Kochsalzlösung (PBS; 0,15 mol/l, pH 7,2) und Essigsäure (0,5 mol/l, pH 2,5) gewaschen. Vor der Verwendung wurde das Adsorbens mit dem 3fachen Gelvolumen PBS äquilibriert. Ausbeute: ca. 20 ml Peptid-Sepharose.

Auf die gleiche Weise wurde das wie in Beispiel 1 hergestellte Tetradecapeptid zum Herstellen eines Immunadsorbens verwendet.

### Beispiel 4

### Isolieren spezifischer Antikörper

100 ml Rohantiserum wurden auf die mit PBS äquilibrierte Oktapeptid-Sepharose (1,5 x 15 cm) aufgetragen und anschließend mit PBS gewaschen, bis die Extinktion bei 280 nm 0,01 betrug. Danach erfolgten Waschschritte mit Kochsalzlösung (1 mol/l, pH 7,0) und Wasser (pH 7,0), wobei jeweils das 3fache Gelvolumen verwendet wurde. Die Elution der Antikörper vom Immunadsorbens erfolgte mit 0,1 mol/l Essigsäure (pH 2,5), die Antikörperlösung wurde mit festem Natriumphosphat auf pH 7,0 eingestellt, konzentriert (Amicon-Membran) und bei -70 °C gelagert. Ausbeute: 35 bis 40 mg Antikörper.

### Beispiel 5

### Testen der immunadsorptiv gewonnenen Antikörper

### a) Herstellen von Antikörper-beschichteten Röhrchen

Die in Beispiel 4 gewonnenen Antikörper wurden mit Tris-Pufferlösung (0,025 mol/l, pH 7,6) auf eine Konzentration von 5 µg/ml verdünnt und durch Adsorption an Polystyrol-Röhrchen immobilisiert. Pro Röhrchen wurden 250 µl Antikörperlösung für 20 Stunden bei 20° C inkubiert, anschließend die Flüssigkeit abgesaugt und die Röhrchen luftdicht verschlossen bei 4° C gelagert.

### b) Durchführen des Enzymimmunoassays (ELISA)

Die zu testenden Proben (Plasma, Serum) wurden mit Inkubationspuffer (0,01 mol/l Tris, 0,01 mol/l EDTA, Heparin (2 U/ml), 0,05 % Tween, pH 7,6) 1:1 verdünnt und jeweils 200 µl pro Röhrchen (siehe Beispiel 5a) 30 min bei 37° C inkubiert. Anschließend wurde die Inkubationslösung entfernt und das Röhrchen zweimal mit je 500 µl Waschlösung (0,02 mol/l Natriumphosphat, 0,05 % Tween, pH 7,6) gewaschen. Anschließend wurden 200 µl Peroxidase-konjugierter Anti-Prothrombin-Antikörper zugefügt und die Röhrchen 30 min bei 37° C inkubiert. Nach Entfernen der Konjugat-Lösung und zweimaligem Waschen wurden 200 µl Substrat-Chromogen-Lösung (Wasserstoffperoxid; o-Phenylendiamin) zugefügt und die Röhrchen bei Raumtemperatur inkubiert. Nach 1/2stündiger Inkubation wurde die Peroxidase mit Schwefelsäure inaktiviert und die Extinktion der Reaktionslösung bei 492 nm bestimmt.

In der nachfolgenden Tabelle sind die Extinktionswerte bei 492 nm in Abhängigkeit von der Plasma- bzw. Serumverdünnung im Vergleich zu der Extinktion eines Röhrchen ohne Plasma oder Serum dargestellt.

**Tabelle 1**

| Verdünnungsstufe | OD₄₉₂/30 min |
|---|---|
| Plasma | |
| 1 : 10 | 0.13 |
| 1 : 100 | 0.12 |
| 1 : 1000 | 0.11 |
| 1 : 10000 | 0.11 |

| Serum | |
|---|---|
| 1 : 10 | 3.72 |
| 1 : 100 | 3.71 |
| 1 : 1000 | 2,81 |
| 1 : 10000 | 0.97 |
| Puffer-Leerwert | 0.045 |

### c) Durchführen eines Enzymimmunoassays (ELISA) zur Bestimmung von in vitro-gebildeten Fragmenten F₂/F₁₊₂.

In einem weiteren Experiment wurde die Spezifität der Antikörper gegen die Fragmente F₂/F₁₊₂ überprüft. Mit Citratlösung antikoaguliertes Plasma wurde mit Calcium-Chlorid-Lösung rekalzifiziert (Endkonzentration von Calciumchlorid: 0.025 mol/l). Zu verschiedenen Zeiten wurden Aliquote entnommen und die Reaktion durch Zugabe von EDTA (0,1 mol/l), Antithrombin III (3 IU/ml) und Heparin (5 IU/ml) abgestoppt. Die Proben wurden mit Inkubationspuffer 1:1 verdünnt und mit dem ELISA getestet.

Die Tabelle gibt die Ergebnisse wieder:

**Tabelle 2**

| Zeit (min.) | OD₄₉₂/39 min. |
|---|---|
| 0 | 0,18 |
| 3 | 0,30 |
| 6 | 0,32 |
| 12 | 0,58 |
| 15 | 1,20 |
| 18 | 1,69 |
| 21 | 1,88 |
| 25 | 1,96 |
| Pufferwert | 0,045 |

Die Ergebnisse zeigen, daß die Fragmente F₂/F₁₊₂ auf diese Weise quantitativ gemessen werden können: während der Rekalzifizierungsreaktion steigt die Konzentration der Fragmente F₂/F₁₊₂ mit zunehmender Zeit an.

Die erfindungsgemäßen Peptide, die eine Aminosäuresequenz aufweisen, die ganz oder teilweise der Aminosäuresequenz von Prothrombin entspricht und antigen ist, induzieren somit die bindungsspezifischen Antikörper gegen die jeweiligen im Peptid vorhandenen antigenen Determinanten. Diese spezifischen Antikörper können anschließend durch Immunadsorption an Peptide mit der gleichen antigenen Determinante gereinigt werden. Die Verwendung synthetischer Peptide hat den wesentlichen Vorteil, daß absolut reine Antigene zur Immunisierung eingesetzt werden, so daß im resultierenden Immunserum keinerlei Kreuzreaktion mit anderen Proteinen oder anderen Teilen des Prothrombinmoleküls auftreten kann. Erfindungsgemäß wird bevorzugt ein Peptid, das die Faktor Xa-Spaltstelle enthält verwendet. Mit einem Antikörper gegen dieses Peptid lassen sich nur gespaltene Prothrombinmoleküle nachweisen, da im intakten nativen Prothrombin diese Sequenz der Antikörpererkennung nicht zugänglich ist. Die Messung der Menge gebundenen Antikörpers erlaubt einen direkten Rückschluß auf die Konzentration freigesetzter Prothrombinfragmente F₂/F₁₊₂ und damit eine Aussage über den Aktivierungsgrad des Prothrombins.

## Patentansprüche

1. Peptide, die Aminosäurensequenzen aufweisen, die teilweise dem durch die F Xa-Spaltung von Prothrombin entstehenden carboxyterminalen Ende der Fragmente F₂/F₁₊₂ entsprechen, dadurch gekennzeichnet, daß sie die Aminosäuresequenz H-Gly-Asp-Glu-Glu-Gly-Val-Trp-Cys-Tyr-Val-Ala-Gly-Lys-Pro-Gly-Asp-Phe-Gly-Tyr-Cys-Asp-Leu-Asn-Tyr-Cys-Glu-Glu-Ala-Val-Gln-Glu-Glu-Thr-Gly-Asp-Gly-Leu-Asp-Glu-Asp-Ser-Asp-Glu-Glu-Arg-Ala-Ile-Glu-Gly-Arg-OH ganz oder teilweise mindestens aber die vier carboxyterminalen Aminosäuren enhalten.

2. Peptid nach Anspruch 1, dadurch gekennzeichnet, daß es die Aminosäuresequenz H-Leu-Asp-Glu-Asp-Ser-Asp-Glu-Glu-Arg-Ala-Ile-Glu-Gly-Arg-OH, H-Glu-Glu-Arg-Ala-Ile-Glu-Gly-Arg-OH oder H-Cys(SH)-Leu-Asp-Glu-Asp-Ser-Asp-Glu-Glu-Arg-Ala-Ile-Glu-Gly-Arg hat.

3. Peptide nach Anspruch 1, dadurch gekennzeichnet, daß sie gentechnisch oder durch chemische Synthese hergestellt werden.

4. Peptid nach Anspruch 1, dadurch gekennzeichnet, daß es an einen unlöslichen polymeren Träger oder Keyhole Limpet Hemocyanin, Albumin oder Ovalbumin gebunden ist.

5. Verwendung der Peptide nach Anspruch 1 zum Immunisieren eines Tieres, bevorzugt eines Kaninchens und zur Reinigung von spezifischen Antikörpern gegen das genannte Peptid mittels Immunadsorption.

6. Verwendung eines Polypeptides nach Anspruch 5, dadurch gekennzeichnet, daß das Peptid die Aminosäuresequenz H-Glu-Glu-Arg-Ala-Ile-Glu-Gly-Arg-OH hat.

7. Antikörper gegen Peptide nach Anspruch 1, dadurch gekennzeichnet, daß sie durch Immunisieren eines Tieres mit einem Peptid nach mindestens einem der Ansprüche 1 bis 4 und anschließende Reinigung über Immunadsorption nach mindesten einem der Ansprüche 5 oder 6 erhalten worden sind.

8. Antikörper nach Anspruch 7, dadurch gekennzeichet, daß sie spezifisch mit einem Peptid nach Anspruch 1 reagieren.

9. Antikörper nach Anspruch 8, dadurch gekennzeichnet, daß sie spezifisch für die Bindung an die Prothrombinfragmente F₂/F₁₊₂ sind und daß sie an einen Träger gekoppelt sind.

10. Verfahren zur Bestimmung von Prothrombinfragment F₁₊₂/F₂ unter Verwendung von Antikörpern nach mindestens einem der Ansprüche 7 bis 9, dadurch gekennzeichnet, daß an den spezifischen Antikörper die Prothombinfragmente F₂/F₁₊₂ gebunden werden, durch einen zweiten Antikörper detektiert werden und daß die Menge des gebundenen zweiten Antikörpers meßbar ist.

11. Verwendung von Antikörpern nach Anspruch 10, dadurch gekennzeichnet, daß an den zweiten Antikörper ein Markerenzym, bevorzugt Peroxydase, gebunden ist.

## Claims

1. Peptides which have amino acid sequences which partly correspond to the carboxyl-terminal end of the fragments F₂/F₁₊₂ resulting from the F Xa cleavage of prothrombin, and which contain the amino acid sequence H-Gly-Asp-Glu-Glu-Gly-Val-Trp-Cys-Tyr-Val-Ala-Gly-Lys-Pro-Gly-Asp-Phe-Gly-Tyr-Cys-Asp-Leu-Asn-Tyr-Cys-Glu-Glu-Ala-Val-Gln-Glu-Glu-Thr-Gly-Asp-Gly-Leu-Asp-Glu-Asp-Ser-Asp-Glu-Glu-Arg-Ala-Ile-Glu-Gly-Arg-OH, in whole or in part, but at least the four carboxyl-terminal amino acids.

2. A peptide as claimed in claim 1, which has the amino acid sequence H-Leu-Asp-Glu-Asp-Ser-Asp-Glu-Glu-Arg-Ala-Ile-Glu-Gly-Arg-OH, H-Glu-Glu-Arg-Ala-Ile-Glu-Gly-Arg-OH or H-Cys(SH)-Leu-Asp-Glu-Asp-Ser-Asp-Glu-Glu-Arg-Ala-Ile-Glu-Gly-Arg.

3. Peptides as claimed in claim 1, which are prepared by genetic manipulation or by chemical synthesis.

4. A peptide as claimed in claim 1, which is bound to an insoluble polymeric carrier or keyhole limpet hemocyanin, albumin or ovalbumin.

5. The use of the peptides as claimed in claim 1 for the immunization of an animal, preferably of a rabbit, and for the purification of specific antibodies against the said peptide by means of immunoadsorption.

6. The use of a polypeptide as claimed in claim 5, wherein the peptide has the amino acid sequence H-Glu-Glu-Arg-Ala-Ile-Glu-Gly-Arg-OH.

7. Antibodies against peptides as claimed in claim 1, which have been obtained by immunization of an animal with a peptide as claimed in at least one of claims 1 to 4 and subsequent purification by immunoadsorption as claimed in at least one of claims 5 and 6.

8. Antibodies as claimed in claim 7, which react specifically with a peptide as claimed in claim 1.

9. Antibodies as claimed in claim 8, which are specific for binding to the prothrombin fragments F₂/F₁₊₂ and are coupled to a carrier.

10. A method for the determination of prothrombin fragment F₁₊₂/F₂ using antibodies as claimed in at least one of claims 7 to 9, which comprises the prothrombin fragments F₂/F₁₊₂ being bound to the specific antibodies and being detected by a second antibody, and comprises the quantity of the bound second antibody being measurable.

11. The use of antibodies as claimed in claim 10, wherein a marker enzyme, preferably peroxidase, is bound to the second antibody.

## Revendications

1. Peptides comportant des séquences d'aminoacides qui correspondent en partie à l'extrémité carboxy-terminale des fragments F₂/F₁₊₂, résultant de la coupure de la prothrombine par le facteur Xa, caractérisés en ce qu'ils contiennent en partie ou en totalité la séquence d'aminoacides H-Gly-Asp-Glu-Glu-Gly-Val-Trp-Cys-Tyr-Val-Ala-Gly-Lys-Pro-Gly-Asp-Phe-Gly-Tyr-Cys-Asp-Leu-Asn-Tyr-Cys-Glu-Glu-Ala-Val-Gln-Glu-Glu-Thr-Gly-Asp-Gly-Leu-Asp-Glu-Asp-Ser-Asp-Glu-Glu-Arg-Ala-Ile-Glu-Gly-Arg-OH, mais au moins les quatre aminoacides à l'extrémité carboxy-terminale de celle-ci.

2. Peptides selon la revendication 1, caractérisés en ce qu'ils comportent la séquence d'aminoacides H-Leu-Asp-Glu-Asp-Ser-Asp-Glu-Glu-Arg-Ala-Ile-Glu-Gly-Arg-OH, H-Glu-Glu-Arg-Ala-Ile-Glu-Gly-Arg-OH ou H-Cys(SH)-Leu-Asp-Glu-Asp-Ser-Asp-Glu-Glu-Arg-Ala-Ile-Glu-Gly-Arg.

3. Peptides selon la revendication 1, caractérisés en ce qu'ils sont produits par génie génétique ou par synthèse chimique.

4. Peptide selon la revendication 1, caractérisé en ce qu'il est lié à un support polymère insoluble ou à de l'hémocyanine de patelle, de l'albumine ou de l'ovalbumine.

5. Utilisation des peptides selon la revendicaon 1, pour l'immunisation d'un animal, de préférence d'un lapin, et pour la purification d'anticorps spécifiques dirigés contre ledit peptide au moyen d'immunoadsorption.

6. Utilisation d'un polypeptide selon la revendication 5, caractérisé en ce que le peptide comporte la séquence d'aminoacides H-Glu-Glu-Arg-Ala-Ile-Glu-Gly-Arg-OH.

7. Anticorps dirigés contre des peptides selon la revendication 1, caractérisés en ce qu'ils ont été obtenus par immunisation d'un animal à l'aide d'un peptide selon au moins l'une des revendications 1 à 4, et purification subsé-quente par immunoadsorption selon au moins l'une des revendications 5 et 6.

8. Anticorps selon la revendication 7, caractérisés en ce qu'ils réagissent spécifiquement avec un peptide selon la revendication 1.

9. Anticorps selon la revendication 8, caractérisés en ce qu'ils sont spécifiques pour la liaison avec les fragments F₂/F₁₊₂ de la prothrombine et en ce qu'ils sont couplés à un support.

10. Procédé pour la détermination du fragment de prothrombine F₁₊₂/F₂ à l'aide d'anticorps selon au moins l'une des revendications 7 à 9, caractérisé en ce que les fragments de prothrombine F₂/F₁₊₂ sont liés aux anticorps spécifiques, sont détectés au moyen d'un second anticorps, et en ce que la quantité du second anticorps lié est mesurable.

11. Utilisation d'anticorps selon la revendication 10, caractérisée en ce qu'une enzyme de marquage, de préférence la peroxydase, est liée au second anticorps.
